# EUROPEAN PATENT SPECIFICATION

(11) **EP 3 162 891 B1**
(45) Date of publication and mention of the grant of the patent: **11.12.2019**
(21) Application number: 15810916.5
(22) Date of filing: 24.06.2015
(51) Int. Cl.: C12M 3/00, C12N 5/071, C12N 15/09, C08J 7/00, C08G 61/08, C12M 1/24, C12M 1/22, C12M 1/42, C12M 1/00, B32B 27/00

(54) **CULTURED CELL DIFFERENTIATION PROMOTION METHOD AND CULTURED CELL DIFFERENTIATION PROMOTER**
VERFAHREN ZUR FÖRDERUNG DER DIFFERENZIERUNG KULTIVIERTER ZELLEN UND PROMOTOR FÜR DIE DIFFERENZIERUNG KULTIVIERTER ZELLEN
PROCÉDÉ POUR PROMOUVOIR LA DIFFÉRENCIATION DE CELLULES EN CULTURE ET PROMOTEUR DE DIFFÉRENCIATION DE CELLULES EN CULTURE

(30) Priority: 26.06.2014 JP 2014130942; 10.02.2015 JP 2015023740
(43) Date of publication of application: 03.05.2017
(73) Proprietor: Zeon Corporation, Tokyo 100-8246 (JP)
(72) Inventor: ICHIMURA, Naoya, Tokyo 100-8246 (JP); HIRANO, Takaaki, Tokyo 100-8246 (JP)
(74) Representative: Maiwald Patent- und Rechtsanwaltsgesellschaft mbH
(86) International application number: PCT/JP2015/068153
(87) International publication number: WO 2015/199117

(56) References cited:
- WO-A1-01/88100
- WO-A1-2006/093207
- WO-A1-2009/099152
- JP-A- 2008 048 653
- JP-A- 2011 115 152
- US-A1- 2002 151 487
- NAVAKANTH R. GANDAVARAPU ET AL: "Osteogenic differentiation of human mesenchymal stem cells on [alpha]5 integrin binding peptide hydrogels is dependent on substrate elasticity", BIOMATERIALS SCIENCE, vol. 2, no. 3, 1 January 2014 (2014-01-01) , pages 352-361, XP055423396, GB ISSN: 2047-4830, DOI: 10.1039/C3BM60149H
- SARAH B. ANDERSON ET AL: "The performance of human mesenchymal stem cells encapsulated in cell-degradable polymer-peptide hydrogels", BIOMATERIALS., vol. 32, no. 14, 1 May 2011 (2011-05-01), pages 3564-3574, XP055422976, GB ISSN: 0142-9612, DOI: 10.1016/j.biomaterials.2011.01.064
- LOBER, A. ET AL.: 'Monolithic polymers for cell cultivation, differentiation, and tissue engineering.' ANGEW. CHEM. INT. ED. vol. 47, no. 47, 2008, ISSN 1433-7851 pages 9138 - 9141, XP055248610
- RAO, V.N. ET AL.: 'Norbornene-derived poly-D- lysine copolymers as quantum dot carriers for neuron growth.' BIOMACROMOLECULES vol. 13, no. 9, 2012, ISSN 1525-7797 pages 2933 - 2944, XP055248613
- WIERINGA, P. ET AL.: 'Nanotopography induced contact guidance of the F11 cell line during neuronal differentiation: a neuronal model cell line for tissue scaffold development.' NANOTECHNOLOGY vol. 23, no. 27, 2012, ISSN 0957-4484 pages 1 - 14, XP020225604

## Description

### TECHNICAL FIELD

The present invention relates to a method for promoting the differentiation of cultured cells, and a cultured cell differentiation promoter.

### BACKGROUND ART

In recent years, regenerative medicine that utilizes pluripotent cells and stem cells (e.g., ES cells and iPS cells) has attracted attention. Pluripotent cells and stem cells are undifferentiated cells that have not differentiated. According to regenerative medicine, these undifferentiated cells are caused to differentiate into the desired internal organ, tissue, or cells, and the patient is treated using the resulting internal organ, tissue, or cells. For example, skin regenerative medicine and cosmetic treatment that utilize the stem cells collected from the epidermal tissue of the patient have been implemented.

The epidermis of the skin includes basal cells, prickle cells, granular cells, and horny cells sequentially from the derma to the surface of the skin. The basal cells sequentially differentiate into prickle cells, granular cells, and horny cells, and are finally removed in the form of scurf.

According to skin regenerative medicine, the basal cells and the prickle cells are collected from the outermost epidermal tissue of the skin of the subject, and cultured in a culture vessel so as to grow in layers while performing a differentiation-inducing treatment so that the outermost cells (outermost cell layer) differentiate into granular cells, and then differentiate into horny cells. The cells (cultured epidermis) obtained by sequentially growing basal cells, prickle cells, granular cells, and horny cells in layers by means of the differentiation-inducing treatment are transplanted into the skin of the subject.

A dish, a flask, or the like that is made of polystyrene that has been subjected to a hydrophilization treatment has been widely used as a culture vessel when performing the differentiation-inducing operation.

It is known that the basal cells and the prickle cells that form the epidermis differentiate into granular cells and horny cells in an environment in which the calcium concentration increases.

The biosynthesis of proteins (e.g., keratin, involucrin, and loricrin) that form the horny cells occurs when the granular cells differentiate into horny cells. Therefore, it is important that the biosynthesis of these proteins occur within the cultured cells when producing cultured epidermis. Note that whether or not the biosynthesis of these proteins has occurred within the cultured cells can be determined by detecting mRNA that corresponds to each protein.

Skin regenerative medicine technology has advanced as described above. However, since a known cultured epidermis production method takes time and effort, there may be a case where the production of cultured epidermis fails to meet the deadline for transplantation surgery, or a considerable cost is required in proportion to the time and effort required to produce the cultured epidermis.

Therefore, a method that can further promote the differentiation of cultured cells has been desired.

A method that promotes differentiation into horny cells using a fermented milk product obtained using lactic acid bacteria is known. Patent Literature 1 discloses that the amount of mRNA of keratin (i.e., epidermal cell differentiation marker) increases when a fermented milk product obtained using *Lactobacillus helveticus* (i.e., lactic acid bacteria) is added to a culture medium that is used to culture human normal epidermal cells. Patent Literature 1 also discloses that oral intake of the fermented milk product promotes the hornification of the epidermis of the skin.

However, since the fermented milk product includes lactic acid bacteria, it is not realistic to mix the fermented milk product into a culture medium that is used when culturing cells used for regenerative medicine.

A method that promotes the differentiation of the epidermal cells using a cherry extract is also known. Patent Literature 2 discloses that the mRNA production with respect to keratin 10 and profilaggrin (i.e., important components during the differentiation process) is promoted by supplying the cherry extract to the epidermal cells. However, keratin 10 and profilaggrin are synthesized in the prickle cells that correspond to the initial stage and the middle stage of the epidermal cell differentiation process, and the cherry extract does not promote differentiation into the horny cells. Moreover, the technology disclosed in Patent Literature 2 is intended for an external preparation such as a cosmetic preparation, and it is difficult to apply the finding disclosed in Patent Literature 2 directly to the promotion of differentiation of epidermal cells used for regenerative medicine.

### CITATION LIST

### PATENT LITERATURE

Patent Literature 1: WO2006/137513 (US2010/0166877)
Patent Literature 2: JP-A-2012-167048

### SUMMARY OF INVENTION

### TECHNICAL PROBLEM

The invention was conceived in view of the above situation. An object of the present invention is to provide a method that can promote the differentiation of cultured cells, and a cultured cell differentiation promoter.

### SOLUTION TO PROBLEM

The inventors conducted extensive studies in order to solve the above problem. As a result, the inventors found that the amount of mRNA (i.e., differentiation marker) increases when the differentiation target cells are cultured while bringing the differentiation target cells into contact with an alicyclic structure-containing polymer. This finding has led to the completion of the invention.

Several aspects of the invention provide the following method for promoting the differentiation of cultured cells (see (1) and (2)) and for the use of a norbornene-based polymer formed article as a cultured cell differentiation promoter wherein the formed article is a member that forms part or the entirety of a culture vessel (see (3)).
(1) A method for promoting the differentiation of cultured cells including bringing a norbornene-based polymer formed article into contact with cells that are being cultured, wherein the formed article is a member that forms part or the entirety of a culture vessel, and
   wherein the norbornene-based polymer is selected from a hydrogenated ring opening polymer of a norbornene-based monomer and a hydrogenated ring opening polymer of a norbornene-based monomer and a monomer that can undergo ring-opening copolymerization with the norbornene-based monomer.
(2) The method for promoting the differentiation of cultured cells according to (1), wherein the cells that are being cultured are epidermal cells.
(3) Use of a norbornene-based polymer formed article as a cultured cell differentiation promoter,
   wherein the formed article is a member that forms part or the entirety of a culture vessel, and
   wherein the norbornene-based polymer is selected from a hydrogenated ring opening polymer of a norbornene-based monomer and a hydrogenated ring opening polymer of a norbornene-based monomer and a monomer that can undergo ring-opening copolymerization with the norbornene-based monomer.

### ADVANTAGEOUS EFFECTS OF INVENTION

The invention thus provides a method that can promote the differentiation of cultured cells, and the use of a norbornene-based polymer formed article as a cultured cell differentiation promoter wherein the formed article is a member that forms part or the entirety of a culture vessel.

### BRIEF DESCRIPTION OF DRAWINGS

FIG. 1 is a graph illustrating a loricrin expression level.
FIG. 2 is a graph illustrating a GATA3 expression level.

### DESCRIPTION OF EMBODIMENTS

The cells used in connection with the invention are not particularly limited as long as the cells can differentiate. Examples of the cells that can differentiate include pluripotent cells; various stem cells such as mesenchymal stem cells; various precursor cells; cells that have differentiated from an ectoderm, a mesoderm, or an endoderm, and have not reached a terminal differentiation state; and the like. For example, epidermal cells that are used to regenerate the epidermis of the skin are preferable as the cells that can differentiate. The epidermal cells may be epidermal basal cells that come in contact with the derma, prickle cells that are situated on the side of the surface of the skin with respect to the basal cells, or granular cells that are situated on the side of the surface of the skin with respect to the prickle cells. It is preferable to use epidermal cells collected from the skin treatment target patient.

A liquid medium is normally used when culturing the cells.

A liquid medium that has a pH buffer action, has an osmotic pressure suitable for the cells, includes nutritional ingredients for the cells, and does not have toxicity to the cells, is normally used as the liquid medium.

Examples of a component that has a pH buffer action include Tris hydrochloride, a phosphate, a carbonate, and the like.

The osmotic pressure of the liquid medium is normally adjusted using an aqueous solution that includes potassium ions, sodium ions, calcium ions, glucose, and the like at an adjusted concentration so that the osmotic pressure of the liquid medium is almost equal to that of the cells. Specific examples of such an aqueous solution include physiological saline such as phosphate buffered saline, Tris-buffered saline, and HEPES-buffered saline; a Ringer's solution such as Ringer's lactate solution, Ringer's acetate solution, and Ringer's bicarbonate solution; and the like.

Examples of the nutritional ingredients for the cells include an amino acid, nucleic acid, a vitamin, a mineral, and the like.

A commercially-available product such as RPMI-1640, HAM, α-MEM, DMEM, EMEM, F-12, F-10, and M-199 may be used as the liquid medium.

An additive may be added to the liquid medium. Examples of the additive include an inducer such as a protein, a low-molecular-weight compound having differentiation-inducing activity, a mineral, a metal, a vitamin component, and the like. It is preferable to add an additive that induces differentiation to the liquid medium.

Examples of the additive that induces differentiation include a ligand, an agonist, and an antagonist that act on a cell surface acceptor; a ligand, an agonist, and an antagonist that act on a nuclear receptor; an extracellular matrix such as collagen and fibronectin; part of the extracellular matrix, and a compound that imitates the extracellular matrix; a component that acts on a protein that is involved in a cell signaling pathway; a component that acts on a primary or secondary metabolism enzyme within the cells; a component that affects intranuclear or intramitochondrial gene expression; DNA and RNA that can be introduced into the cells in combination with a virus vector or the like; and the like.

These additives may be used either alone or in combination.

The cell culture conditions are not particularly limited. The cell culture conditions may be appropriately determined corresponding to the cells and the object.

For example, the cells may be cultured using a humidified thermostat that contains carbon dioxide at a concentration of about 5%, and is maintained at a constant temperature within the range from 20°C to 37°C.

The alicyclic structure-containing polymer formed article used in connection with the invention is obtained by forming an alicyclic structure-containing polymer so as to have an arbitrary shape. According to the claimed invention the alicyclic structure-containing polymer is a norbornene-based polymer selected from a hydrogenated ring-opening polymer of a norbornene-based monomer and a hydrogenated ring-opening polymer of a norbornene-based monomer and a monomer that can undergo ring-opening copolymerization with the norbornene-based monomer.

The term "alicyclic structure-containing polymer" used herein refers to a resin that includes an alicyclic structure in either or both of the main chain and the side chain. It is preferable that the alicyclic structure-containing polymer include an alicyclic structure in the main chain from viewpoint of mechanical strength, heat resistance, and the like. According to the claimed invention the alicyclic structure-containing polymer is a norbornene-based polymer selected from a hydrogenated ring-opening polymer of a norbornene-based monomer and a hydrogenated ring-opening polymer of a norbornene-based monomer and a monomer that can undergo ring-opening copolymerization with the norbornene-based monomer.

Examples of the alicyclic structure include a saturated cyclic hydrocarbon (cycloalkane) structure, an unsaturated cyclic hydrocarbon (cycloalkene) structure, and the like. It is preferable that the alicyclic structure-containing polymer include a cycloalkane structure or a cycloalkene structure from the viewpoint of mechanical strength, heat resistance, and the like. It is most preferable that the alicyclic structure-containing polymer include a cycloalkane structure. According to the claimed invention the alicyclic structure-containing polymer is a norbornene-based polymer selected from a hydrogenated ring-opening polymer of a norbornene-based monomer and a hydrogenated ring-opening polymer of a norbornene-based monomer and a monomer that can undergo ring-opening copolymerization with the norbornene-based monomer.

The number of carbon atoms included in the alicyclic structure is not particularly limited, but is normally 4 to 30, preferably 5 to 20, and more preferably 5 to 15. When the number of carbon atoms included in the alicyclic structure is within the above range, the alicyclic structure-containing polymer exhibits mechanical strength, heat resistance, and formability in a highly balanced manner. According to the claimed invention the alicyclic structure-containing polymer is a norbornene-based polymer selected from a hydrogenated ring-opening polymer of a norbornene-based monomer and a hydrogenated ring-opening polymer of a norbornene-based monomer and a monomer that can undergo ring-opening copolymerization with the norbornene-based monomer.

The content of an alicyclic structure-containing repeating unit in the alicyclic structure-containing polymer may be appropriately selected taking account of the intended use, but is normally 30 wt% or more, preferably 50 wt% or more, and still more preferably 70 wt% or more. If the content of the alicyclic structure-containing repeating unit in the alicyclic structure-containing polymer is too low, the alicyclic structure-containing polymer may exhibit poor heat resistance. A repeating unit that may be included in the alicyclic structure-containing polymer in addition to the alicyclic structure-containing repeating unit is not particularly limited, and may be appropriately selected taking account of the intended use.

The alicyclic structure-containing polymer claimed herein is (1) a norbornene-based polymer selected from a hydrogenated ring-opening polymer of a norbornene-based monomer and a hydrogenated ring-opening polymer of a norbornene-based monomer and a monomer that can undergo ring-opening copolymerization with the norbornene-based monomer. Further examples include (2) a monocyclic cycloolefin-based polymer, (3) a cyclic conjugated diene-based polymer, (4) a vinyl alicyclic hydrocarbon-based polymer, hydrogenated products of the polymers (1) to (4), and the like. Among these,
a norbornene-based polymer and a hydrogenated product thereof are preferable from the viewpoint of heat resistance, mechanical strength, and the like.

### (1) Norbornene-based polymer

The term "norbornene-based polymer" used herein refers to a polymer that is obtained by polymerizing a norbornene-based monomer (i.e., a monomer that includes a norbornene skeleton). Norbornene-based polymers are roughly classified into a norbornene-based polymer obtained by ring-opening polymerization, and a norbornene-based polymer obtained by addition polymerization.

Examples of the norbornene-based polymer obtained by ring-opening polymerization include a ring-opening polymer of a norbornene-based monomer, a ring-opening polymer of a norbornene-based monomer and a monomer that can undergo ring-opening copolymerization with the norbornene-based monomer, hydrogenated products thereof, and the like. Examples of the norbornene-based polymer obtained by addition polymerization include an addition polymer of a norbornene-based monomer, an addition polymer of a norbornene-based monomer and a monomer that can undergo addition copolymerization with the norbornene-based monomer, and the like. Among these, a hydrogenated ring-opening polymer of a norbornene-based monomer is preferable from the viewpoint of heat resistance, mechanical strength, and the like.

Examples of the norbornene-based monomer include a bicyclic norbornene-based monomer such as bicyclo[2.2.1]hept-2-ene (trivial name: norbornene), 5-methylbicyclo[2.2.1]hept-2-ene, 5,5-dimethylbicyclo[2.2.1]hept-2-ene, 5-ethylbicyclo[2.2.1]hept-2-ene, 5-ethylidenebicyclo[2.2.1]hept-2-ene, 5-vinylbicyclo[2.2.1]hept-2-ene, 5-propenylbicyclo[2.2.1]hept-2-ene, 5-methoxycarbonylbicyclo[2.2.1]hept-2-ene, 5-cyanobicyclo[2.2.1]hept-2-ene, and 5-methyl-5-methoxycarbonylbicyclo[2.2.1]hept-2-ene; a tricyclic norbornene-based monomer such as tricyclo[4.3.0^{1,6}.1^{2,5}]deca-3,7-diene (trivial name: dicyclopentadiene), 2-methyldicyclopentadiene, 2,3-dimethyldicyclopentadiene, and 2,3-dihydroxydicyclopentadiene; a tetracyclic norbornene-based monomer such as tetracyclo[4.4.0.1^{2,5}.1^{7,10}]-3-dodecene (tetracyclododecene), tetracyclo[4.4.0.1^{2,5}.1^{7,10}]-3-dodecene, 8-methyltetracyclo[4.4.0.1^{2,5}.1^{7,10}]-3-dodecene, 8-ethyltetracyclo[4.4.0.1^{2,5}.1^{7,10}1-3-dodecene, 8-ethylidenetetracyclo[4.4.0.1^{2,5}.1^{7,10}]-3-dodecene, 8,9-dimethyltetracyclo[4.4.0.1^{2,5}.1^{7,10}]-3-dodecene, 8-ethyl-9-methyltetracyclo[4.4.0.1^{2,5}.1^{7,10}]-3-dodecene, 8-ethylidene-9-methyltetracyclo[4.4.0.1^{2,5}.1^{7,10}]-3-dodecene, 8-methyl-8-carboxymetfiyltetracyclo[4.4.0.1^{2,5}.1^{7,10}]-3-dodecene, 7,8-benzotricyclo[4.3.0.1^{2,5}]dec-3-ene (trivial name: methanotetrahydrofluorene (also referred to as 1,4-methano-1,4,4a,9a-tetrahydrofluorene)), 1,4-methano-8-methyl-1,4,4a,9a-tetrahydrofluorene, 1,4-methano-8-chloro-1,4,4a,9a-tetrahydrofluorene, and 1.4-methano-8-bromo-1,4,4a,9a-tetrahydrofluorene; and the like.

Examples of the monomer that can undergo ring-opening copolymerization with the norbornene-based monomer include a monocyclic cycloolefin-based monomer such as cyclohexene, cycloheptene, cyclooctene, 1,4-cyclohexadiene, 1,5-cyclooctadiene, 1,5-cyclodecadiene, 1,5,9-cyclododecatriene, and 1,5,9,13-cyclohexadecatetraene.

These monomers may be substituted with one substituent, or may be substituted with two or more substituents. Examples of the substituent include an alkyl group, an alkylene group, an aryl group, a silyl group, an alkoxycarbonyl group, an alkylidene group, and the like.

Examples of the monomer that can undergo addition copolymerization with the norbornene-based monomer include an α-olefin-based monomer having 2 to 20 carbon atoms, such as ethylene, propylene, 1-butene, 1-pentene, and 1-hexene; a cycloolefin-based monomer such as cyclobutene, cyclopentene, cyclohexene, cyclooctene, and tetracyclo[9.2.1.0^{2,10}.0^{3,8}]tetradeca-3,5,7,12-tetraene (also referred to as "3a,5,6,7a-tetrahydro-4,7-methano-1H-indene); a non-conjugated diene-based monomer such as 1,4-hexadiene, 4-methyl-1,4-hexadiene, 5-methyl-1,4-hexadiene, and 1,7-octadiene; and the like.

Among these, an α-olefin-based monomer is preferable, and ethylene is more preferable.

These monomers may be substituted with one substituent, or may be substituted with two or more substituents. Examples of the substituent include an alkyl group, an alkylene group, an aryl group, a silyl group, an alkoxycarbonyl group, an alkylidene group, and the like.

A ring-opening polymer of a norbornene-based monomer, or a ring-opening polymer of a norbornene-based monomer and a monomer that can undergo ring-opening copolymerization with the norbornene-based monomer, may be obtained by polymerizing the monomer component in the presence of a known ring-opening polymerization catalyst. Examples of the ring-opening polymerization catalyst include a catalyst that includes a halide of a metal (e.g., ruthenium or osmium), a nitrate or an acetylacetone compound, and a reducing agent, and a catalyst that includes a halide or an acetylacetone compound of a metal (e.g., titanium, zirconium, tungsten, or molybdenum), and an organoaluminum compound.

A hydrogenated ring-opening polymer of a norbornene-based monomer may normally be obtained by adding a known hydrogenation catalyst that includes a transition metal (e.g., nickel or palladium) to a solution including the ring-opening polymer, and hydrogenating the carbon-carbon unsaturated bonds of the ring-opening polymer.

An addition polymer of a norbornene-based monomer, or an addition polymer of a norbornene-based monomer and a monomer that can undergo addition copolymerization with the norbomene-based monomer, may be obtained by polymerizing the monomer component in the presence of a known addition polymerization catalyst. Examples of the addition polymerization catalyst include a catalyst that includes a titanium, zirconium, or vanadium compound and an organoaluminum compound.

### (2) Monocyclic cycloolefin-based polymer

Examples of the monocyclic cycloolefin-based polymer include an addition polymer of a monocyclic cycloolefin-based monomer (e.g., cyclohexene, cycloheptene, or cyclooctene).

### (3) Cyclic conjugated diene-based polymer

Examples of the cyclic conjugated diene-based polymer include a 1,2-addition polymer or a 1,4-addition polymer of a cyclic conjugated diene-based monomer (e.g., cyclopentadiene or cyclohexadiene), a hydrogenated product thereof, and the like.

### (4) Vinyl alicyclic hydrocarbon polymer

Examples of the vinyl alicyclic hydrocarbon polymer include a polymer of a vinyl alicyclic hydrocarbon-based monomer (e.g., vinylcyclohexene or vinylcyclohexane), and a hydrogenated product thereof; a hydrogenated product obtained by hydrogenating the aromatic ring of a polymer of a vinyl aromatic-based monomer (e.g., styrene or α-methylstyrene); and the like. The vinyl alicyclic hydrocarbon polymer may be a copolymer of the above monomer and an additional monomer that is copolymerizable with the above monomer.

The molecular weight of the alicyclic structure-containing polymer is not particularly limited. The polystyrene-equivalent weight average molecular weight of the alicyclic structure-containing polymer determined by gel permeation chromatography using a cyclohexane solution (or a toluene solution when the polymer does not dissolve in cyclohexane) is normally 5,000 or more, preferably 5,000 to 500,000, more preferably 8,000 to 200,000, and particularly preferably 10,000 to 100,000. When the weight average molecular weight of the alicyclic structure-containing polymer is within the above range, the alicyclic structure-containing polymer exhibits mechanical strength and formability in a highly balanced manner.

The glass transition temperature of the alicyclic structure-containing polymer may be appropriately selected taking account of the intended use. The glass transition temperature of the alicyclic structure-containing polymer is normally 50 to 300°C, preferably 100 to 280°C, more preferably 115 to 250°C, and still more preferably 130 to 200°C. When the glass transition temperature of the alicyclic structure-containing polymer is within the above range, the alicyclic structure-containing polymer exhibits heat resistance and formability in a highly balanced manner.

Note that the glass transition temperature of the alicyclic structure-containing polymer refers to a value measured in accordance with JIS K 7121.

The alicyclic structure-containing polymers described above may be used either alone or in combination.

An additive that is normally used for a thermoplastic resin material, such as a soft polymer, an antioxidant, a UV absorber, a light stabilizer, a near-infrared absorber, a release agent, a coloring agent (e.g., dye and pigment), a plasticizer, an antistatic agent, and a fluorescent whitening agent, may be added to the alicyclic structure-containing polymer in an amount that is normally employed.

A polymer other than the soft polymer (hereinafter referred to as "additional polymer") may be mixed with the alicyclic structure-containing polymer. The additional polymer is normally mixed with the alicyclic structure-containing polymer in a ratio of 200 parts by weight or less, preferably 150 parts by weight or less, and more preferably 100 parts by weight or less, based on 100 parts by weight of the alicyclic structure-containing polymer.

If the ratio of the additive or the additional polymer is too large based on the alicyclic structure-containing polymer, the effect of promoting the differentiation of cells may decrease. Therefore, it is preferable to add (mix) the additive and the additional polymer within such a range that the properties of the alicyclic structure-containing polymer are not impaired.

The additive and the additional polymer may be mixed with the alicyclic structure-containing polymer using an arbitrary method as long as the additive and the additional polymer are sufficiently dispersed in the alicyclic structure-containing polymer. The additive and the additional polymer may be added in an arbitrary order. Examples of the mixing method include a method that mixes (kneads) the resin in a molten state using a mixer, a single-screw kneader, a twin-screw kneader, a roll, a Brabender, an extruder, or the like, a method that dissolves the resin in an appropriate solvent to effect dispersion, and removes the solvent using a coagulation method, a casting method, or a direct drying method, and the like.

When the resin is mixed (kneaded) using a twin-screw kneader, the resulting mixture (kneaded product) is normally extruded in the shape of a rod in a molten state, and cut (pelletized) to have an appropriate length using a strand cutter.

A forming method used when forming the alicyclic structure-containing polymer may be arbitrarily selected corresponding to the shape of the alicyclic structure-containing polymer formed article that is brought into contact with cells. Examples of the forming method include an injection forming method, an extrusion method, a cast forming method, an inflation forming method, a blow forming method, a vacuum forming method, a press forming method, a compression forming method, a rotational forming method, a calendering method, a roll forming method, a cutting method, a spinning method, and the like. Note that these methods may be used in combination, and a post-treatment such as stretching may optionally be performed after forming.

The resulting formed article is used as the cultured cell differentiation promoter according to the invention.

The shape of the alicyclic structure-containing polymer formed article is not particularly limited. The alicyclic structure-containing polymer formed article may have a plate-like shape, a powdery shape, a particulate shape, a string-like shape, a sheet-like shape, or the like. The surface of the alicyclic structure-containing polymer formed article may be flat or irregular. The alicyclic structure-containing polymer formed article may be a hollow formed article. A plurality of formed articles that differ in shape may be combined through an adhesive or the like, or may be combined without using an adhesive or the like, to form another formed article.

The alicyclic structure-containing polymer formed article claimed is a member that forms part or the entirety of a culture vessel (e.g., dish, plate, bag, tube, scaffold, cup, and jar fermenter). Further examples are a part of a culture apparatus (e.g., stirring blade, stirring bar, baffle, and connection tube), a culture tool used for culture operation (e.g., pipette, stirring device, filter, and cell scraper), and the like, as long as the alicyclic structure-containing polymer formed article can be brought into contact with the cells.

When implementing the method according to the invention, there is a tendency that the cells that are being cultured survive in the culture medium in a suspended state irrespective of whether the cells are adherent cells or suspension cells. Therefore, the alicyclic structure-containing polymer formed article can be brought into contact with the cells in various ways.

When implementing the invention, it is preferable to sterilize the formed article before bringing the formed article into contact with the cultured cells. The sterilization method is not particularly limited. The sterilization method may be selected from sterilization methods that are normally employed in the medical field (e.g., a heating method such as a high-pressure steam method and a dry heat method; a radiation method that applies radiation such as γ-rays or an electron beam, and an irradiation method that applies high-frequency waves; a gas method that brings a gas such as ethylene oxide gas (EOG) into contact with the sterilization target; and a filtration method that utilizes a sterilization filter) corresponding to the shape of the formed article and the cells to be cultured.

The surface of the formed article may also be subjected to a treatment (e.g., plasma treatment, corona discharge treatment, ozone treatment, and UV irradiation treatment) other than a sterilization treatment that is normally applied to a culture vessel.

The alicyclic structure-containing polymer formed article (i.e., the cultured cell differentiation promoter according to the invention) may be brought into contact with the cultured cells using an arbitrary method corresponding to the vessel shape of the cultured cell differentiation promoter. For example, the alicyclic structure-containing polymer formed article may be brought into contact with the cultured cells using a method that cultures the cells in a culture medium into which the alicyclic structure-containing polymer formed article (cultured cell differentiation promoter) is mixed; a method that cultures the cells in a culture vessel that is formed using the alicyclic structure-containing polymer; a method that cultures the cells using a culture tool that is formed using the alicyclic structure-containing polymer; or the like. These methods may be used in combination.

Since cells have a signaling capability, all of the cultured cells need not necessarily come in contact with the alicyclic structure-containing polymer formed article, and the cultured cells need not necessarily come in contact with the alicyclic structure-containing polymer formed article during the entire culture period. Note that it is preferable that the contact time be as long as possible in order to compensate for a decrease in effect with the passing of time.

The temperature at which the alicyclic structure-containing polymer formed article is brought into contact with the cultured cells is not particularly limited as long as the cells can be grown.

### EXAMPLES

The invention is further described below by way of examples. Note that the invention is not limited to the following examples.

### Production Example 1

A culture dish (culture tool) having a bottom diameter of 3 cm was produced as described below using an alicyclic structure-containing polymer (norbornene-based polymer ("ZEONEX (registered trademark) 790R" (hydrogenated norbornene-based ring-opening polymer) manufactured by Zeon Corporation)). Note that the resulting culture dish is hereinafter referred to as "790R dish".

The 790R dish was produced using an injection forming method, and sterilized by a gas method using ethylene oxide gas (EOG).

### Example 1

Human epidermal keratinocytes (for producing a human epidermis model) used for a three-dimensional skin regeneration kit manufactured by Japan Tissue Engineering Co., Ltd. were seeded onto the 790R dish at a cell density of 1.25×10⁴ cells/cm², and cultured for 6 days in a CO₂ incubator. The resulting sample is hereinafter referred to as "790R dish sample".

### Comparative Example 1

Cells were cultured in the same manner as in Example 1, except that a cell culture dish manufactured by Corning ("Falcon (registered trademark) 353001") (hereinafter referred to as "polystyrene dish") was used instead of the 790R dish. The resulting sample is hereinafter referred to as "polystyrene dish sample".

### Comparative Example 2

Cells were cultured (simultaneously with Example 1 or Comparative Example 1) while inducing differentiation under the conditions described below.

Epidermal cells were seeded onto the polystyrene dish at a cell density of 1.25×10⁴ cells/cm², and cultured in a CO₂ incubator in parallel to the above culture sample. Calcium chloride (calcium agent) was added to the polystyrene dish when 1 day had elapsed from the start of culture, and the cells were cultured for 5 days. The resulting sample is hereinafter referred to as "calcium-added polystyrene dish sample".

### Evaluation of differentiation state of cells

In Example 1 and Comparative Examples 1 and 2, a differentiation marker that was expressed in the cells, was analyzed as described below in order to evaluate the differentiation state of the cells. Loricrin that is a protein that forms horny cells (i.e., the terminally differentiated state of epidermal cells) was used as an index with respect to the differentiation marker.

After completion of the culture period, cells were collected from each sample, and the amount of loricrin mRNA contained in the cells was quantitatively determined by real-time PCR (see below). The mRNA of GAPDH was used as an internal standard.

RNA was extracted from the cells using a QuickGene RNA cultured cell kit S (manufactured by Kurabo Industries, Ltd.). The RNA was converted to DNA through a reverse transcription reaction using a PrimerScript RTase (manufactured by Takara Bio Inc.). Real-time PCR using the DNA prepared through the reverse transcription reaction as a template was performed using a PCR-CFX96 system (manufactured by BioRad).

The culture experiment was repeated twice, and the amount of loricrin mRNA contained in the 790R dish sample (Example 1) and the amount of loricrin mRNA contained in the polystyrene dish sample (Comparative Example 1) were calculated using the average expression level of loricrin mRNA with respect to the amount (=1.0) of loricrin mRNA contained in the calcium-added polystyrene dish sample (Comparative Example 2). The results are shown in Table 1.

**TABLE 1**

| | Loricrin mRNA expression level (relative value) |
|---|---|
| Example 1 | 1.10 |
| Comparative Example 1 | 0.087 |
| Comparative Example 2 | 1.00 |

As is clear from a comparison between Comparative Examples 1 and 2, the differentiation of the cells was promoted through the addition of the calcium agent, and the amount of loricrin mRNA significantly increased.

On the other hand, the amount of loricrin mRNA contained in the 790R dish sample (Example 1) was larger than the amount of loricrin mRNA contained in the calcium-added polystyrene dish sample (Comparative Example 2), although the calcium agent that promotes the differentiation of the cells was not added. Therefore, it was confirmed that the differentiation of the cultured cells was promoted by bringing the alicyclic structure-containing polymer formed article into contact with the cultured cells.

### Example 2

In order to evaluate the effect of the culture tool (cultured cell differentiation promoter) according to the invention when the cells are cultured in the presence of a calcium agent, the cells were cultured in the same manner as in Example 1, except that the calcium agent was added to the culture medium when 1 day had elapsed from the start of culture, and the expression level of loricrin mRNA (differentiation marker) was quantitatively determined.

The amount of loricrin mRNA contained in the calcium-added 790R dish sample (Example 2) was 2.379 with respect to the amount (=1.0) of loricrin mRNA contained in the calcium-added polystyrene dish sample (Comparative Example 2). It was thus confirmed that the amount of loricrin mRNA significantly increased in Example 2.

These results suggest that it is possible to increase the secretion amount of intracellular components necessary for differentiation by bringing the cells into contact with the alicyclic structure-containing polymer formed article, and reduce the time required for the differentiation-inducing operation.

Loricrin is the main component that forms the stratum corneum, and maintains the multilayer structure consisting of horny cells and lipids. It is considered that the epidermis becomes finer when the expression of loricrin occurs sufficiently.

### Production Examples 2 to 11

A culture dish having a bottom diameter of 3 cm was produced in the same manner as in Production Example 1, except that "APEL (registered trademark) APL6013T" (norbomene-based polymer) manufactured by Mitsui Chemicals Inc., "TOPAS (registered trademark) 6013" (norbornene-based polymer) manufactured by Polyplastics Co., Ltd., "ARTON (registered trademark) D4540" (norbornene-based polymer) manufactured by JSR Corporation, polycarbonate, poly(methyl methacrylate) (PMMA), polystyrene, polyethylene, polypropylene, or polyethylene terephthalate (PET) was used instead of ZEONEX (registered trademark) 790R. The resulting dishes are hereinafter referred to as "APEL dish", "TOPAS dish", "ARTON dish", "polycarbonate dish", "PMMA dish", "polystyrene dish", "polyethylene dish", "polypropylene dish", and "PET dish", respectively.

The 790R dish was subjected to a plasma treatment to obtain a plasma surface-treated 790R dish.

### Example 3

Human epidermal keratinocytes (for producing a human epidermis model) were seeded onto the 790R dish at a cell density of 1.25×10⁴ cells/cm² (N=1), and cultured at 37°C for 6 days in a 5% CO₂ atmosphere.

### Examples 4 to 7

Cells were cultured in the same manner as in Example 3, except that the plasma surface-treated 790R dish, the APEL dish (comparative), the TOPAS dish (comparative), and the ARTON dish were respectively used instead of the 790R dish.

### Comparative Examples 3 to 9

Cells were cultured in the same manner as in Example 3, except that a dish manufactured by Becton, Dickinson and Company (FALCON (registered trademark), polystyrene dish, plasma-treated) (FALCON dish), the polycarbonate dish, the PMMA dish, the polystyrene dish, the polyethylene dish, the polypropylene dish, and the PET dish were respectively used instead of the 790R dish.

In Examples 3 to 7 and Comparative Examples 3 to 9, RNA was extracted from the cells, RNA was converted to DNA, and the expression of mRNA was analyzed in the same manner as in Example 1.

Note that the loricrin gene was used as the quantitative determination target gene, the GAPDH gene was used as the internal standard gene, and the amplified signal of the loricrin gene was normalized by dividing the amplified signal of the loricrin gene by the amplified signal of the GAPDH gene.

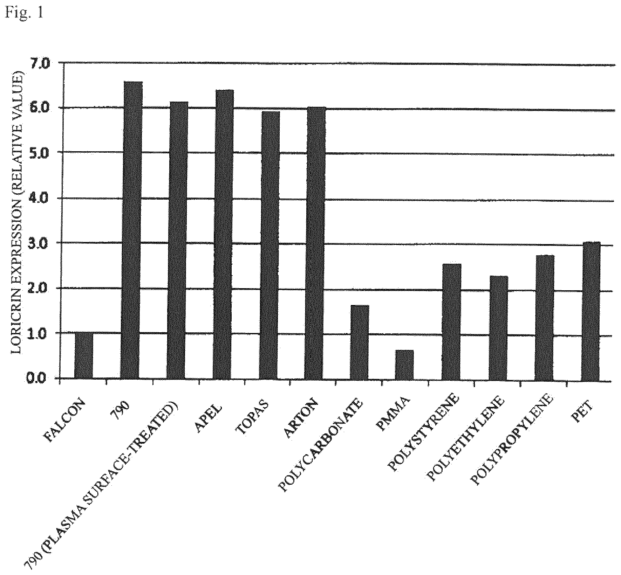

FIG. 1 illustrates the expression level of the loricrin gene in each sample with respect to the expression level (=1) of the loricrin gene in the cell sample cultured using the FALCON dish (Comparative Example 3).

As illustrated in FIG. 1, when the dishes formed of the alicyclic structure-containing polymers other than ZEONEX (registered trademark) 790R were used, the expression level of the loricrin gene was higher than that achieved using the FALCON dish by a factor of about 6. On the other hand, when the dishes formed of the materials other than the alicyclic structure-containing polymer were used, the expression level of the loricrin gene was higher than that achieved using the FALCON dish by only a factor of about 2 to 3. It was thus confirmed that the alicyclic structure-containing polymer has an effect of promoting the induction of differentiation of keratinocytes.

In order to evaluate the difference in the expression state of a differentiation-inducing gene in keratinocytes under culture conditions that induce the differentiation of keratinocytes, the expression of the GATA3 gene that induces differentiation into keratinocytes was determined (compared) using the cells cultured in Examples 3 to 7 and Comparative Examples 3 to 9.

The GATA3 gene was used as the quantitative determination target gene, the GAPDH gene was used as the internal standard gene, and the amplified signal of the GATA3 gene was normalized by dividing the amplified signal of the GATA3 gene by the amplified signal of the GAPDH gene.

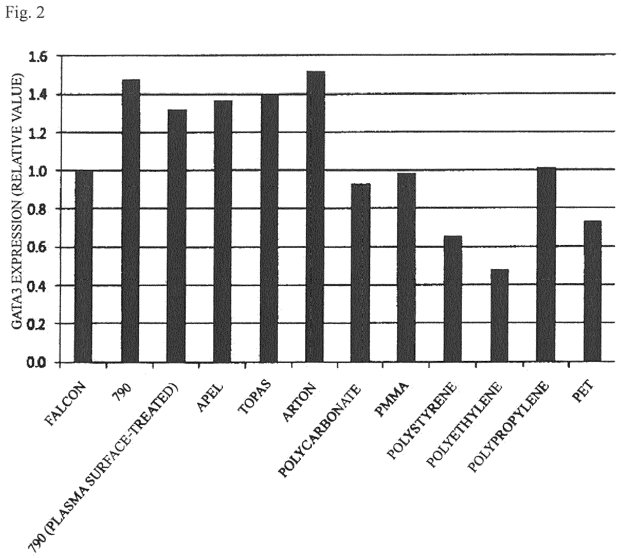

FIG. 2 illustrates the expression level of the GATA gene in each sample with respect to the expression level (=1) of the GATA3 gene in the cell sample cultured using the FALCON dish (Comparative Example 3).

As illustrated in FIG. 2, when the polycarbonate dish, the PMMA dish, or the polypropylene dish was used, the expression level of the GATA3 gene was almost equal to that achieved when the FALCON dish was used. When the polystyrene dish, the polyethylene dish, or the PET dish was used, the expression level of the GATA3 gene was lower than that achieved when the FALCON dish was used (i.e., the induction of differentiation was suppressed).

When the dish made of each alicyclic structure-containing polymer was used, the expression level of the GATA3 gene was higher than that achieved when the FALCON dish was used by a factor of 1.3 to 1.5.

When the 790 dish was used, the expression level of the GATA3 gene was higher than that achieved when the FALCON dish was used by a factor of 1.3 or more irrespective of whether or not the 790 dish was subjected to a plasma surface treatment. It was thus confirmed that the alicyclic structure-containing polymer has an effect of promoting the induction of differentiation of keratinocytes.

## Claims

1. A method for promoting the differentiation of cultured cells comprising bringing a norbornene-based polymer formed article into contact with cells that are being cultured,
wherein the formed article is a member that forms part or the entirety of a culture vessel, and
wherein the norbornene-based polymer is selected from a hydrogenated ring-opening polymer of a norbornene-based monomer and a hydrogenated ring-opening polymer of a norbornene-based monomer and a monomer that can undergo ring-opening copolymerization with the norbornene-based monomer.

2. The method for promoting the differentiation of cultured cells according to claim 1, wherein the cells that are being cultured are epidermal cells.

3. Use of a norbornene-based polymer formed article as a cultured cell differentiation promoter
wherein the formed article is a member that forms part or the entirety of a culture vessel, and
wherein the norbornene-based polymer is selected from a hydrogenated ring-opening polymer of a norbornene-based monomer and a hydrogenated ring-opening polymer of a norbornene-based monomer and a monomer that can undergo ring-opening copolymerization with the norbornene-based monomer.

## Patentansprüche

1. Verfahren zur Förderung der Differenzierung kultivierter Zellen umfassend in Kontakt bringen eines Polymerformkörpers auf Norbornen-Basis mit Zellen, die kultiviert werden,
wobei der Formkörper ein Element ist, das einen Teil oder eine Gesamtheit eines Kulturgefäßes bildet, und
wobei das Polymer auf Norbornen-Basis ausgewählt ist aus einem hydrierten Ringöffnungspolymer aus einem Monomer auf Norbornen-Basis und einem hydrierten Ringöffnungspolymer aus einem Monomer auf Norbornen-Basis und einem Monomer, das einer Ringöffnungscopolymerisation mit dem Monomer auf Norbornen-Basis unterzogen werden kann.

2. Verfahren zur Förderung der Differenzierung kultivierter Zellen gemäß Anspruch 1, wobei die Zellen, die kultiviert werden, epidermale Zellen sind.

3. Verwendung eines Polymerformkörpers auf Norbornen-Basis als Promotor für die Differenzierung kultivierter Zellen,
wobei der Formkörper ein Element ist, das einen Teil oder eine Gesamtheit eines Kulturgefäßes bildet, und
wobei das Polymer auf Norbornen-Basis ausgewählt ist aus einem hydrierten Ringöffnungspolymer aus einem Monomer auf Norbornen-Basis und einem hydrierten Ringöffnungspolymer aus einem Monomer auf Norbornen-Basis und einem Monomer, das einer Ringöffnungscopolymerisation mit dem Monomer auf Norbornen-Basis unterzogen werden kann.

## Revendications

1. Procédé pour favoriser la différenciation de cellules cultivées, comprenant la mise en contact d'un article façonné en polymère à base de norbornène avec des cellules en cours de culture,
dans lequel l'article façonné est un élément qui forme une partie ou la totalité d'un récipient de culture, et
dans lequel le polymère à base de norbornène est choisi parmi un polymère hydrogéné obtenu par ouverture de cycle d'un monomère à base de norbornène et un polymère hydrogéné obtenu par ouverture de cycle d'un monomère à base de norbornène et d'un monomère qui peut subir une copolymérisation par ouverture de cycle avec le monomère à base de norbornène.

2. Procédé pour favoriser la différenciation de cellules cultivées selon la revendication 1, dans lequel les cellules qui sont cultivées sont des cellules épidermiques.

3. Utilisation d'un article façonné en polymère à base de norbornène en tant que promoteur de différenciation d'une cellule cultivée,
dans lequel l'article façonné est un élément qui forme une partie ou la totalité d'un récipient de culture, et
dans lequel le polymère à base de norbornène est choisi parmi un polymère hydrogéné obtenu par ouverture de cycle d'un monomère à base de norbornène et un polymère hydrogéné obtenu par ouverture de cycle d'un monomère à base de norbornène et d'un monomère qui peut subir une copolymérisation par ouverture de cycle avec le monomère à base de norbornène.
